# EUROPEAN PATENT APPLICATION

(11) **EP 2 946 722 A1**
(43) Date of publication of application: **25.11.2015**
(21) Application number: 14169077.6
(22) Date of filing: 20.05.2014
(51) Int. Cl.: A61B 5/00, A61B 5/08

(54) **Portable electronic device for breath sampling**

(71) Applicant: Sensirion AG, 8712 Stäfa (CH)
(72) Inventor: Hoehne, Felix, 8712 Staefa (CH)

(57) **Abstract**

A portable electronic device and a related methods are described using an portable electronic device, preferably with telecommunication capabilities to allow for data and/or voice communication via private or public networks, with an integrated gas sensor (12,21) sensitive to hydrogen and/or methane and/or carbon monoxide, and an opening (107) for a contactless delivery of a breath sample of a user to the sensor and elements ( configured to store a time sequence of data representative of a plurality of hydrogen and/or methane concentrations and optionally a correlation and testing unit for correlating the time sequence of concentration measurements with a time sequence of events relating to the food intake of the user and for performing tests, in particular test for food intolerances or cigarette consumption, on the correlated data.

## Description

### FIELD OF THE INVENTION

The present invention relates to a portable electronic device such as a mobile phone, tablet and the like with an integrated gas sensor configured to detect the presence of breath components in a sample of a user's breath.

### BACKGROUND OF THE INVENTION

Portable or mobile devices originally introduced as mobile phones or electronic agendas become more and more ubiquitous. As the processing power of their internal processors grows and equally the bandwidth for communication with stationary processors, such portable devices take on more and more the role of multi-purpose tools available to consumers and specialist users alike.

It has been recognized that portable devices can benefit from the presence of sensors capable of providing a chemical analysis of materials brought into contact or the vicinity of the device. Whilst there are many possible applications for such sensors, it suffices to consider for example the analysis of air surrounding the portable device. Such an analysis can be useful for multiple purposes such as testing for hazardous gases, breath analysis for general medical purposes or driving fitness, and the like.

Specialized devices for testing the breath for ketones are well known and can for example be found in the United States patent application 2008/0077037.

Recently progress towards portable devices has been reported in the NTT DOCOMO Technical Journal Vol. 14, No. 1, 51 - 57. However, the described device remains a specialized equipment, which can communicated with remote servers only via a separate mobile phone.

A detection integrated in a mobile device is briefly referred to in the United States patent no. 8280436 B2 purporting to use infrared spectrophotometers, electrochemical fuel cells or other gas analysers.

The co-owned US patent application serial number 14/161,114 with the title "Portable electronic device with Ketone sensor" with a filing date of January 22, 2014 (claiming priority of the European patent application 13405025.1 with a filing date of January 31, 2013) describes the integration of a tubeless breath analyzer into a portable device. However the device is used for the detection of ketones in a sample of breath.

It is known to use a hydrogen breath test (or HBT) as a clinical medical diagnosis for people with irritable bowel syndrome, and common food intolerances. The test is simple, non-invasive, and is performed after a short period of fasting (typically 8-12 hours). Even though the test is normally known as a "Hydrogen Breath Test" some physicians may also test for methane in addition to hydrogen. Many studies have shown that some patients (approximately 35% or more) do not produce hydrogen but actually produce methane. Some patients produce a combination of the two gases. It requires to take readings every 15, 30 or 60 minutes for two to three hours. If the patient produces methane then the parts per million for the methane typically rises 12 ppm over the lowest preceding value to be considered positive. These and similar tests are typically performed as a clinical test under supervision and using specialized equipment.

In the published patent application US 2012/186999 it is suggested to use an electrochemical sensor with a metal species capable of catalyzing the oxidation of hydrogen and/or methane in the detection and quantification of hydrogen and/or methane in exhaled breath, for example as a means of diagnosing lactose malabsorption or lactose intolerance.

The international patent application WO 01/87352 describes various test procedures for lactose and fructose intolerance. Test for analyzing smoking habits based on the amount of carbon monoxide in the breath are also known.

Humidity sensors for mobile applications are described for example in the published United States patent application US 2012/231841 and ways of manufacturing miniaturized sensors as MEMS devices with a CMOS connections and circuitry are described for example in the published international patent applications WO 2012/100362 A1 or WO 95/19563.

In view of the prior art it is an object of the present invention to provide a portable apparatus and a testing methods for lactose and/or fructose intolerance and related disorders which are easily deployable outside of clinics and simple to use.

### SUMMARY OF THE INVENTION

Hence, according to a first aspect of the invention, there is provided a portable electronic device, preferably with telecommunication capabilities to allow for data and/or voice communication via private or public networks, with an integrated gas sensor sensitive to hydrogen and or methane, and an opening for a contactless delivery of a breath sample of a user to the sensor and elements configured to store a time sequence of data representative of a plurality of hydrogen (H2) and/or methane (CH4) and/or carbon monoxide (CO) concentrations. The device can be configured to automatically requests breath samples of the user over an extended testing period which may extend beyond one hour, or even three or more hours, or eight hours or may extend over the duration of the full day or even a week. In contrast to existing portable devices, these elements of the invention enable the monitoring a testing of trends in the measured concentration data.

The sensor includes preferably a correlation and testing unit for correlating the time sequence of concentration measurements with a time sequence of events relating to the food intake of the user and for performing tests, in particular test for food intolerances, or a test for smoking habits on the correlated data and related disorders and regular monitoring of these conditions.

The test is performed on a subset for the full set of the measured data are stored, including at least two, preferably three or more of the stored concentration measurements to allow for trend analysis and/or gradient analysis to be performed by the portable device. In particular, the test can include a test for an increase in the amount of hydrogen in the breath of more than 15 - 20 ppm above a baseline, which in turn can also be derived from a sequence of samples.

The device preferably includes further sensors, in particular one or more contextual sensors (271,272,273) and/or other gas, temperature and humidity sensors (13,24) with such sensors being configured to provide input to the event log either automatically or in combination with an input of the user. The sensors, particularly several gas sensors, can be implemented as independent sensors each with a dedicated driver and readout electronic circuit or as sensor pixels where two or more sensors of the same or similar type share driver and/or read-out electronic circuit.

In a preferred embodiment of the invention, the time sequence of events includes events relating to user activity other than food intake, such as sport activities or events relating to cigarette consumption.

Preferably the device includes a display driver and display for displaying signals or information representative of the result of the testing and/or related warnings.

The representation or display can be qualitatively in form of, for example, a color signal indicating to the user the presence or absence of a food intolerance by a change of color or the display of a respective message.

The sensor is preferably implemented using a CMOS process or a CMOS compatible process such that leads connecting the metal oxide sensor to the control circuit use metal layers of the CMOS substrate. In a preferred variant of this embodiment, a heater used to heat the metal oxide gas sensor is also connected to metal layers of the CMOS substrate. Using such an integrated design, it is possible to reduce the size of the sensor and the integrated control circuit to less the 5mm by 5mm, preferably even less than 2mm by 2mm.

The reduced size facilitates the integration into a portable electronic device, such that in a preferred embodiment the sensor and the control circuit can be enclosed in a housing having an air duct with an opening to the exterior of the housing connecting the gas sensor to the outside and with the total area of the opening being sufficiently small to act as restriction to diffusion, such as 10 square millimeters or 3.14 square millimeters or even less. Such integration is seen as important as for general purpose devices it is often not acceptable to have external tubes connected to the housing of the device.

The integration into the housing of a portable device with data communication capabilities can be used to communicate measured raw data or processed data from the sensor to be sent to remote locations for further processing. Based on preference settings selected by the user, the transmittal can be automated by allowing the processing unit of the device to access the registers or memory location at which the sensor data are stored. The processing at a remote location can be used to improve the measurement or generate user alerts to be transmitted back the device.

As the device is preferably tubeless, i.e. lacks a dedicated tube or mouth piece for breath sampling and/or means to correct for the dilution, breath duration and breath intensity.

The portable device can be a smart phone, a handheld computer, a laptop, an electronic reader, a tablet computer, a game controller, a pointing device, a photo or a video camera, digital music player, wrist watch, key fob, head set or a computer peripheral. Its housing is typically a shell of metal, glass, or plastic material and can be assembled as a unibody or from several parts. Enclosed in the housing are typically processors, drivers for parts such as screens, antennae, cameras, microphones and speakers as well as batteries to provide power to the device and its parts. A screen is typically arranged as a part of the housing or mounted behind a transparent window of the housing.

The above and other aspects of the present invention together with further advantageous embodiments and applications of the invention are described in further details in the following description and figures.

### BRIEF DESCRIPTION OF THE FIGURES

FIG. 1A is a perspective view of a portable electronic device;
FIG. 1B is a schematic view into part of the housing of the device of Fig. 1A;
FIG. 2 is a block diagram with components of a portable device in accordance with an example of the invention;
FIG. 3 illustrates processing steps in accordance with an example of the invention; and
FIG. 4 shows an example of a combined or time correlated concentration log and event log.

### DETAILED DESCRIPTION

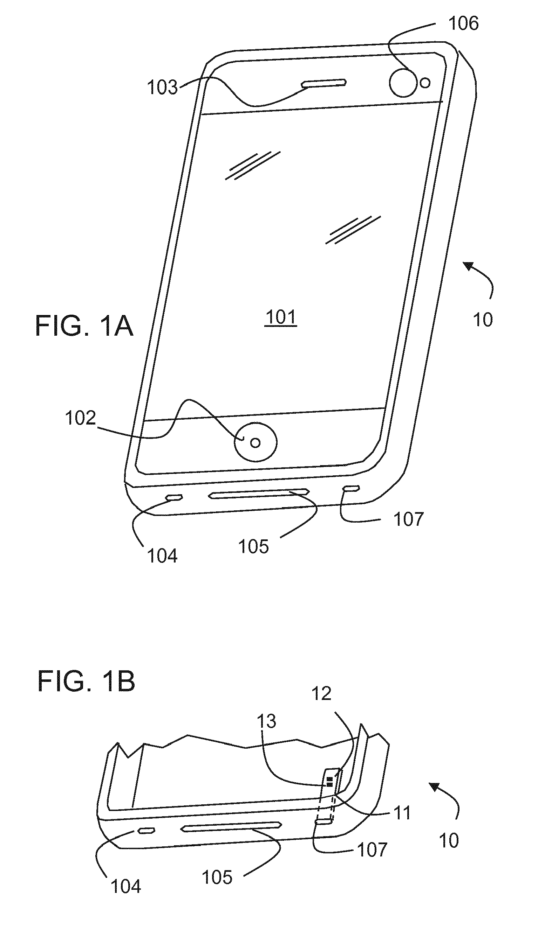

The device of FIG. 1A is a portable electronic device such as a mobile phone. The housing 10 of the mobile phone includes a front side with a screen 101 and elements like buttons 102 to let a user interact with the phone. Also shown on the front side is an opening 103 for a loudspeaker. Further openings 104, 105 are located at a lower side wall of the housing 10. It is well known to mount components like microphones and loudspeakers behind such openings. The phone includes one or two cameras 106, and internally additional sensors (not shown) such as location sensors or GPS, and acceleration and orientation sensors in a manner well known.

Another opening 107 is located at the lower side wall. As shown in FIG. 1B the opening 107 is linked to a tubular duct 11 passing through the interior of the housing. A gas sensor 12 configured to detect the concentration of hydrogen and/or methane in a gaseous sample and a humidity sensor 13 are both mounted along the duct 11 such that the sensitive areas of both sensors are essentially exposed air of the same composition entering the duct through the opening 107.

The gas sensor 12 can be for example a single sensor, such as a metal oxide type sensor or an electrochemical sensor as described for example in US 2012/186999 referred to above, or an array or assembly of several sensors or sensor pixels sharing the electronic circuit for sensing and read-out of results. The gas sensors can be either of the same type of metal oxide sensors but with a different sensing material or, either alternatively or in addition, sensors based on a different sensing principle. The sensor can also be coated with a layer of catalyst such as transition metals and their oxides.

The actual size and shape of the duct 11 depends on the volume available and the nature of the gas sensor 12 and the humidity sensor 13 can vary, but given the physical constraints of portable mobile devices the area of the opening is typically in the range of less than 10 square millimeters and in the present example actually about less than 3.1 square millimeters.

The opening 107 is used to capture a sample of a user's breath. It should be noted that the device operates without a dedicated mouthpiece or tube and hence without contact between the lips or model of the user and any of the device. This aspect of the device is considered important as it allows for repeated measurements while maintaining basic, yet sufficient hygiene standards. To ensure that the sample is representative of the exhaled breath corrective measures such as described for example in the above cited US patent application serial number 14/161,114 may be required including a correction for breath dilutions and/or for the size of the opening. To perform a breath test the user may be requested to change the orientation of the device such that the opening 107 is in line with the direction of the exhaled breath.

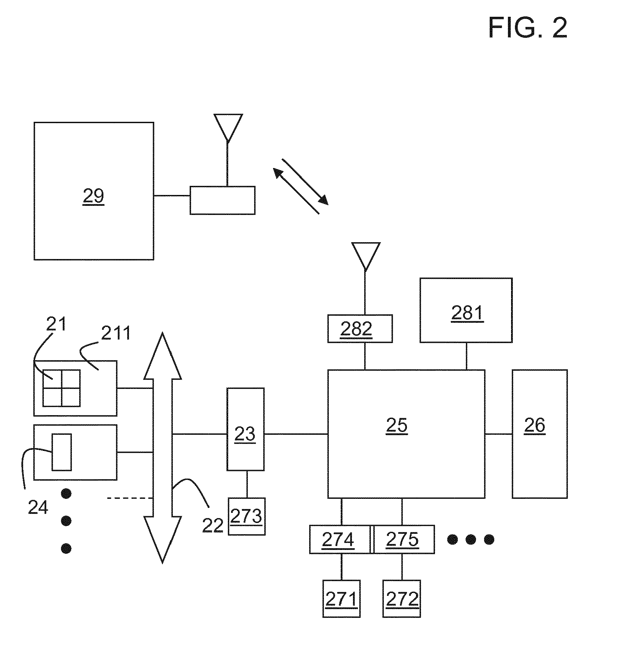

Fig. 2 shows a block diagram view illustrating the most important components of the portable device from a functional point of view. In particular, the device includes a gas sensor 21 (the physical layout of which is described when referring to FIG.1 above) integrated as part of a CMOS substrate 211 which has CMOS circuitry to control the basic functions and the basic readout of the sensor. The CMOS circuit can include for example the driver to switch the sensor and his heater on or off as well as A/D converters and amplifiers and an I2C bus controller to exchange data on an I2C bus 22. The I2C bus connects the sensors with a sensor hub 23. A further humidity and temperature sensor 24 is also linked to the I2C bus 22.

The sensor hub 23 provides a control and processing unit for more complex control and read-out functions of the gas sensor 21 based on signals sent to or extracted from, respectively, the on-chip CMOS circuitry.

Further control and read-out function, such as time control and user prompts or alerts, can also be performed by the central processing unit (CPU)25 of the portable device, which in turn has read/write access to a memory 26, which can include static or volatile memory or both as known in the art. The memory 26 typically stores the operating system of the device and can also be used to store programs specific to the operation of the sensors of the portable device.

In addition to the specific sensors as described above, the CPU is also connected to one or more sensors 271, 272, 273 which typically operate independently of the operations of the gas sensor. These sensors provide other functionality or capabilities of the portable device, thus creating an independently useable output of the portable device with no direct connection to the gas sensor 21. One of these for the purpose of the present invention contextual sensors is for example the camera 271 or the microphone 272 also shown as the camera 106 and the microphone 104 of FIG. 1. Other examples are location, acceleration and orientation sensors. The contextual sensors 271, 272, 273 communicate with the CPU using their own interface units 274, 275, or via the sensor hub 23, respectively, which operate typically in complete independence of the gas sensor 21.

The device includes further well known input/output units 281 such as a touch sensitive display, virtual or physical keyboards and gesture tracking devices etc. The portable device as shown has a telecommunication circuit 282 comprising an antenna, driver circuits and encoding and decoding units as are well known in the art. Using such a telecommunication circuit and a network interface unit NIU 282, the device can connect to remote data processing and storage facilities 29 as shown.

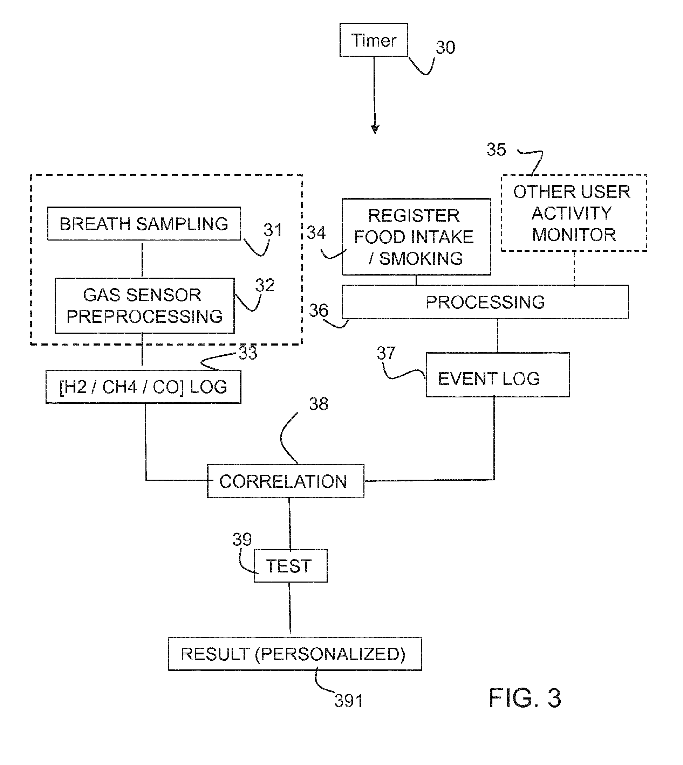

Exemplary operation modes of the above device are described in the following making further reference to the steps of the flowchart of FIG. 3. The functional elements described below can be implemented using the above hardware elements such as the CPU in combination with an application program.

A measurement in accordance with an example of the present invention includes the use of a timer 30 which provides a relative or absolute time to the other components involved in the measurement. It is assumed that a user wishing to perform a lactose or fructose test initializes the device by selecting the appropriate functionality. Once a time line is established through the timer 30, the user is requested to perform a breath analyzing step 31 as described above at regular or irregular intervals. Depending on the nature of the gas sensor, the timer 30 coordinates the sampling with an initialization of the gas sensor. In case of a metal oxide sensor, the sensor is for example heated using internal heating elements to its operating temperature in preparation for each measurement.

The fraction of the exhaled breath entering the duct 11 and thus contacting the gas sensor is analyzed in the preprocessing step 32. The measured concentration is stored together with the time related information representing the time of the measurement in the concentration log 33.

The device is further configured to monitor the food intake 34 of the user in parallel with the repeated breath sampling and logging of the results of the concentration measurements. In addition to monitoring food intake 34 the device can also be configured to monitor other user activity. The monitoring of food intake 34 or other activities 35 can be based on requesting user input or at least partly automated using the sensors of the device as described above. Further details and examples of the monitoring steps 34, 35 and their processing 36 are described below.

The result of the monitoring steps 34, 35 and the processing 36 is stored together with the time related information representing the time of the food intake or activity in an event log 37.

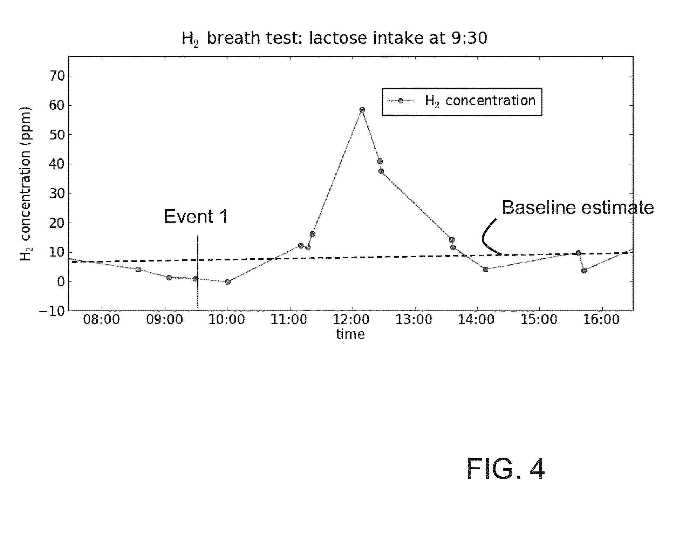

A graphical representation of the concentration log 33 and the event log 37 is shown in FIG. 4. The values and times represented in the graph are typically stored in machine-readable form in the memory 26. The graph assumes that a user has chosen to commence a test in the morning. During the course of the day the application issues requests for breath sampling the results of which are represented as dots. At 9:30 AM a lactose intake is registered in the event log (Event 1) shown as a bar with the height representing an estimate of the amount of lactose. The dashed line represents a baseline estimate for the hydrogen concentration in the user's breath.

Referring again to FIG. 3, the concentration log 33 and the event log 37 are processed together in a correlation unit 38. The correlation unit 38 and the subsequent test unit 39 can be used to store sets of rules and conditions derived from prior knowledge of physiological behavior of the human metabolism. An example of such a rule is the time delay between intake of lactose and the increased production of hydrogen or methane in the human body. A possible test condition of a test as performed by the test unit 39 can be for example the difference between the peak value of the concentration and the baseline.

The result of the test 39 can be used to generate a signal or message for the user making use of user interface facilities as described above. It should be noted that the test and/or its results can be personalized making use of known and appropriately stored conditions of the user.

The basic elements of the example described above can be further refined or alternatively implemented to facilitate and/or automate the monitoring of food intake or other activities. For that inputs can be used from the one or more of the contextual sensor measurements 37. To perform the contextual sensor measurements 37 is per se well known as are all the components and processing steps 38 required to operate the contextual sensors 271,272,273. Their independent functions within the operation of the portable device are well known as such and not considered relevant for the purpose of the present example operations.

For example the camera 271 can be used to let the user record barcodes on food packages, which in turn can be converted by the CPU 25 into a data characterizing the food and ultimately enabling the CPU to determine qualitatively and/or quantitatively lactose intake. The identification of a food product can be used to retrieve further details from the manufacturer or producer or general remote databases using the telecommunication capabilities of the device.

The camera can also be used to acquire image data which in turn can be converted into information about the type and the amount of food shown in the acquired image. The extraction of such information from an image can use any of the known image recognition methods available such as for example described in United States patent no. 8254699 to Google Inc. The output of the image recognition process is a high level description of the content of the image. Using the camera of the device or other wearable cameras, such as cameras available commercially under the trade name Google Glass, linked to the device in an always-on mode and the image recognition process it is possible to automate the registration of food intake at least under controlled circumstances.

In this as in other complex processing steps use can be made not only of the internal processing facilities such as the CPU 25 but also of processing facilities external to the device by transferring inputs as registered by the device via telecommunication circuit and network interface unit NIU 282 to remote data processing and storage facilities 29 as shown in FIG. 2.

In a similar manner the microphone can be used to let a user described the food intake or other activities with words applying for example the speech recognition facilities as are available in modern telecommunication devices.

Other input methods such as keyboard or touch sensitive screens can also be used to register food intake or other events.

All of the above methods of registering food intake or other events can be used separately of each other or concurrently in a complementary manner.

Whilst the registering or monitoring of food intake is an important part of the event log as described above and shown in FIG. 4, further information can be registered relating for example to conditions or status of the user, such as weight, height, age, gender, medical preconditions, smoking habits etc., or environmental parameters such as temperature, humidity, a pressure etc. These further information may either be registered as part of the event log 37 or as part of the correlation unit 38 or the test unit 39 to determine or select the appropriate correlation or test. The information can be static, i.e., constant for at least the duration of the testing, or dynamic, i.e., registered or monitored during the duration of the test. Dynamic information can be stored together with the time marker as provided by the timer 30.

The input of static and dynamic information can be done using any of the available user interface elements of the device. The input can be facilitated and/or automated using the sensors integrated in the device. Hence, environmental parameters can be automatically monitored using for example a temperature sensor, the humidity sensor, or of the global positioning system GPS, where such sensors are part of the device. In a similar manner, the sensors can be used to automatically monitor user status and conditions. As an example, sport related activities can be monitored using GPS or accelerometers without additional user input or interaction.

In another example of dynamic information relevant for the testing of food intolerance is smoking. When equipped with a CO gas sensor, based for example on a SnO2 MOX gas sensor, the device can monitor the amount of CO in the breath sample. Evaluating the trend of such sample can be used to generate alerts to a user. A decline in CO concentration indicates less consumption and may be used to trigger a positive or encouraging feedback to the user attempting to cease smoking while the reverse trend can be used to trigger a warning alert. Such alert can be helpful for example for pregnant women trying to reduce their cigarette consumption.

Apart from being tested through a carbon monoxide gas sensor, a smoking event for the event log 37, i.e., the consumption of a cigarette can be logged by a user or automatically monitored using the other internal sensors such as a using the camera to identify smoke in the environment of the user.

The breath sampling and monitoring for smoking can also be applied as part of the testing for lactose or fructose as described above or any other metabolism test the result of which is known to be altered or distorted if the user smokes during the testing period.

For the purpose of interpreting automated inputs as provided by one or more of the sensors as inputs to the event log or correlation or testing, well established methods in signal processing can be applied. These methods can include any of the known routines for complex data analysis including but not limited to Principal Component Analysis, Linear Discriminant Analysis and the like or neural network based tools such as Selforganizing Maps, Back Propagation, pattern recognition routines etc. For more complex in relation of the inputs use can be made of the telecommunication capabilities 282 of the device and hence of the remote data processing and storage facilities 29 as shown in FIG. 2.

The addition the monitor for user activity can register also other information derived for example through comparison with other users, peer groups, neighborhood. It is also possible that the event log 37, the correlation 38, or the test 39 takes into account information gained by comparison with the breath samples of other users using similar devices and using similar food intake or sharing the same conditions.

Whilst not being necessarily suitable as a replacement for an accurate diagnosis tool, the personalized result 391 output of the device can include information which are supportive or complimentary to a treatment as prescribed by a doctor.

While there are shown and described presently preferred embodiments of the invention, it is to be understood that the invention is not limited thereto but may be otherwise variously embodied and practised within the scope of the following claims. For example, it is possible to use sensors external to the portable device as additional sensors. Such external sensors can be the sensors of a second portable device in the vicinity of the portable device or, more generally, any sensor in the spatial or temporal surrounding of the portable device, the output of which can be linked to provide additional information, for example to populate the event log.

## Claims

**1.** Portable electronic device enclosed in a housing (10) and comprising one or more gas sensor(s) (12,21), a gas sensor and/or general purpose processing unit (211,23,25), data storage (26), a timer (30), input/output units (281), and an opening (107) in the housing connecting the gas sensor(s) (12,21) to the exterior, configured such that a user can deliver a sample of exhaled breath through the opening (107) to the gas sensor(s) without lip contact between any part of the device or housing (10), wherein the gas sensor(s) comprise(s) a hydrogen and/or methane and/or carbon monoxide sensor, and wherein the device is configured to after initialization of a test routine automatically request a plurality of breath samples from the user, and to generate a concentration log (33) representative of results of the plurality measurements together with time information.

**2.** The portable electronic device according to claim 1, comprising a correlation unit (38) configured to time correlate the concentration log with a stored event log (37) of information representative of food intake or cigarette consumption of the user together with time information.

**3.** The portable electronic device according to claim 1 or 2, further comprising a testing unit (39) configured to perform pre-defined testing procedure on the correlated concentration log (33) and the event log.

**4.** The portable electronic device according to 3, wherein testing procedure includes a test for food intolerances, in particular lactose or fructose intolerances, or smoking habits.

**5.** The portable electronic device according to any of the preceding claims 2 to 4, wherein the event log includes events relating to user activities other than food intake.

**6.** The portable electronic device according to any of the preceding claims 2 to 5, comprising further sensors, in particular one or more contextual sensors (271,272,273) and/or gas, temperature and humidity sensors (13,24) and wherein such sensors are configured to provide input to the event log.

**7.** The portable electronic device according to any of the preceding claims, further comprising a telecommunication link (282) to enable a signal exchange with remote processors and/or data basis (29).

**8.** The portable electronic device according to any of the preceding claims, wherein the gas sensor(s) comprise(s) a metal-oxide sensing material.

**9.** The portable electronic device according to any of the preceding claims, wherein the gas sensor(s) is (are) integrated onto a common substrate including CMOS circuitry.

**10.** The portable electronic device according to any of the preceding claims, being selected from a group comprising:
a mobile phone,
a handheld computer,
an electronic reader,
a tablet computer,
a game controller,
a pointing device,
a photo or a video camera,
a digital music player,
an electronic wrist watch,
a headset, and
a computer peripheral.

**10.** A method of operating a portable electronic device with the portable electronic device being enclosed in a housing (10) and comprising one or more gas sensor(s) (12,21) for determining a hydrogen and/or methane concentration and/or a carbon monoxide concentration, a gas sensor and/or general purpose processing unit (211,23,25), data storage (26), a timer (30), input/output units (281), and an opening (107) in the housing connecting the gas sensor(s) (12,21) to the exterior, said method comprising the steps of
- initializing of test routine;
- letting a user deliver during the run of the test routine a plurality of samples of exhaled breath through the opening (107) to the gas sensor(s) without lip contact between any part of the device or housing (10);
- generating a concentration log (33) representative of results of the plurality measurements together with time information.

**11.** The method of claim 10, further comprising the steps of
- generating an event log (37) of information representative of food intake of the user together with time information;
- time correlating (38) the concentration log and the event log-;
- performing one or more predefined tests (39) on the correlated concentration and the event log.

**12.** The method of claim 11, wherein the test is a test for food intolerance, in particular lactose or fructose intolerance, or a test for smoking habits.

**13.** The method of claim 12, wherein the test is a test for food intolerance, in particular lactose or fructose intolerance, correlated with events relating to smoking.

**14.** The method of any of claims 10 to 13, further including the step of generating and displaying the results of the test or related information in a message to the user.

**15.** Set of machine-readable instructions, when stored and executed, configuring the device in accordance with any of the preceding claims.
